# EUROPEAN PATENT APPLICATION

(11) **EP 3 651 181 A1**
(43) Date of publication of application: **13.05.2020**
(21) Application number: 18205179.7
(22) Date of filing: 08.11.2018
(51) Int. Cl.: H01J 35/10, A61B 6/00

(54) **X-RAY SOURCE SYSTEM AND X-RAY IMAGING SYSTEM HAVING A CONVERSION STRUCTURE FOR COMPENSATING CONVERSION EFFICIENCY**

(71) Applicant: Koninklijke Philips N.V., 5656 AG Eindhoven (NL)
(72) Inventor: BEHLING, Rolf Karl Otto, 5656 AE Eindhoven (NL)
(74) Representative: de Haan, Poul Erik

(57) **Abstract**

The present disclosure relates to an X-ray source system configured for successively or concurrently generating different X-ray spectra, which have different cutoff-energies. The system comprises an electron receiving member, which is mounted to be rotatable about a rotation axis. The electron receiving member comprises a conversion structure and a substrate. The system further comprises a voltage generator, which is connected to the electron source and which is configured to control the electron source so that electrons having a first energy and a second energy are generated using different acceleration voltages of the electron beam source. The first energy is greater than the second energy and a ratio of a transmittance for the electrons of the first energy through the conversion structure to a transmittance for the electrons of the second energy through the conversion structure is greater than 1.2.

## Description

### FIELD OF THE INVENTION

The present invention relates to to X-ray systems for generating X-ray spectra of different cutoff-energies and to X-ray computed tomography systems having such an X-ray system.

### BACKGROUND OF THE INVENTION

Known in the prior art are dual-energy computed tomography systems, which use separate energy sets acquired at two different X-ray spectra to examine attenuation properties of matter at different X-ray energies. Such systems have proven to have significant advantages over traditional single energy computed tomography. Specifically, dual energy computed tomography systems allow acquisition of multiple scans of the same subject under the same conditions using two different X-ray spectra. The scans are then used to analyze the material composition of the target. By way of example, soft tissue and similar materials having a relatively low density typically attenuate incident X-rays to a lesser degree compared to body portions or material having a comparatively high absorption of X-rays, such as bone or an iodine contrast agent.

It is appreciated in the art that computer tomography imaging performed at two imaging scans, one at a higher x-ray tube acceleration voltage setting, such as a level between 110 kVp to 150 kVp, and another imaging scan performed at a lower x-ray tube acceleration voltage setting, such as a level between 60 to 100 kVp, provides more information about the materials being scanned than does a single-energy computer tomography imaging scan.

However, it has been shown that the conventional X-ray tubes typically generate the different X-ray spectra at different intensities. The reason for that typically resides in different conversion efficiencies within the electron target for converting the kinetic electron energy into X-rays.

Therefore, a need exists for providing an X-ray source system and an X-ray imaging system, which includes such an X-ray source system, which allow more efficient imaging.

### SUMMARY OF THE INVENTION

Embodiments of the present invention pertain to an X-ray source system configured for successively or concurrently generating different X-ray spectra, which have different cutoff-energies. The system comprises an electron receiving member, which is mounted to be rotatable about a rotation axis. The electron receiving member comprises a conversion structure and a substrate, wherein the conversion structure comprises one or more conversion layers which are directly or indirectly applied to the substrate. The system further comprises an electron source configured to, successively or concurrently, generate one or more beams of electrons which are incident on the conversion structure. The system further comprises a voltage generator, which is in signal communication with the electron source and which is configured to control the electron source so that electrons having a first energy and a second energy are successively or concurrently generated using different acceleration voltages of the electron beam source. Within the electron receiving member, a conversion of a kinetic energy of the electrons into X-rays is suppressed outside of the conversion structure compared to within the conversion structure. The first energy is greater than the second energy and a ratio of a transmittance for the electrons of the first energy through the conversion structure to a transmittance for the electrons of the second energy through the conversion structure is greater than 1.2, or greater than 1.4, or greater than 1.5, or greater than 2, or greater than 2.5, or greater than 3, or greater than 5, or greater than 10.

The X-ray source system may include a housing. The housing may shield against at least a portion of the X-rays, which are generated within the housing. The housing may include an X-ray transmissive portion through which at least a portion of the X-rays exit from the housing. At least a portion of the X-rays which exit from the housing through the X-ray transmissive portion may be directed toward the subject. The electron receiving member and/or at least a portion of the electron source, such as a filament and/or a cathode (in particular a hot cathode or a field emitting cold cathode) may be arranged within the housing. An exterior of the housing may be at atmospheric pressure.

The cut-off energy may be a cut-off energy of a spectrum, which is at least partially formed as bremsstrahlung. The spectrum may further include characteristic radiation (i.e. one or more fluorescence lines) of one or more elements contained in the conversion structure. The electron receiving member may be configured as a rotating anode. However, it is also conceivable, that additionally or alternatively, a gate is arranged in the beam path of the electrons between the electron source and the electron receiving member, which functions as an anode.

An angular speed of the rotation of the electron receiving member about the rotation axis has a value of at least 1,000 r.p.m. (revolutions per minute) or at least 9,000 r.p.m. The angular speed may be less than 20,000 r.p.m. The conversion layer may be directly applied to the substrate. Alternatively, the conversion layers may be applied to the substrate via one or more intermediate layers of the electron receiving member. The intermediate layers may have a function of a interconnecting layer and/or of a diffusion barrier, in particular a molecular diffusion barrier.

The electron source may be configured as a thermionic electron source. The electron source may include a filament, such as a tungsten (W) filament, a hot cathode, such as an impregnated cathode, or a field emitting cold cathode, such as at least one, in particular an array, of carbon nanotubes. The electrons may be emitted from the electron source and guided by an electron optical system of the electron source to the electron receiving member. The electrons may be incident on a surface portion of the electron receiving member which is formed by the conversion structure. The conversion structure includes one or more layers. If the conversion structure includes a plurality of layers, the layers may be adjacent relative to each other or separated from each other by one or more intermediate layers of the electron receiving member. For each of the intermediate layers, the respective intermediate layer may be part of the conversion structure. Alternatively, the respective layer may be part of the electron receiving member without being part of the conversion structure. One or more of the intermediate layers may have the function of an interconnecting layer and/or a diffusion layer.

The X-ray source system may be configured so that the electrons of the first energy and the electrons of the second energy are incident on at least partially overlapping surface regions of the conversion structure or on non-overlapping surface portions.

The X-ray source system may be configured to be a part of a computer tomography system. In particular, the X-ray source system may be a part of a multiple-energy, in particular a dual-energy X-ray tomography system. The X-ray source system may be configured to perform the switching between the generation of the electrons of the first energy and the generation of the electrons of the second energy during a scan of at least a portion of the subject.

The transmittance for the electrons of the first energy and the electrons of the second energy may be measured for an impingement direction at which the electrons of the first energy and the electrons of the second energy are impinging on the conversion structure. The impingement direction of the electrons of the first energy may be different from an impingement direction of the electrons of the second energy. Additionally or alternatively, the electrons of the first energy may be part of an electron beam, which is different from an electron beam which includes the electrons of the second energy.

According to an embodiment, at a portion of the conversion structure, where the electrons of the first and second energies are incident, a thickness of the conversion structure, as measured in a direction parallel to an axis of at least one of the one or more electron beams, is less than 20 micrometer or less than 10 micrometer or less than 7 micrometer. If one or more intermediate layers are disposed between two layers of the conversions structure and these intermediate layers do not form part of the conversion structure, the intermediate layers are not taken into account when determining the thickness of the conversion structure.

According to an embodiment, a difference between the first and the second energy is greater than 4 keV, or greater than 10 keV, or greater than 20 keV, or greater than 30 keV. Additionally or alternatively, the first energy is at least 80 keV, or at least 120 keV, or at least 140 keV.

According to a further embodiment, for each of the one or more layers of the conversion structure, the respective layer comprises one or more elements in a total amount of at least 50 wt% (weight percent) or at least 70 wt%, or at least 80 wt%. In each of the layers, each of the one or more elements of the respective layer has an atomic number of at least 29, or at least 42, or at least 74, or at least 75. The term "total amount" may be defined herein to mean the sum of the amounts of two or more elements. For each pair of the layers, the one or more elements in the first layer may be the same as, or may be at least partially different from, the one or more elements in the second layer.

According to an embodiment, at least a portion of the substrate, within which at least a portion of the electrons are stopped, comprises one or more elements in a total amount of at least 50 wt% or at least 70 wt%, or at least 80 wt%, wherein each of the elements has an atomic number of at most 13. Each of the elements may have an atomic number of at most 6. Each of the elements may have an atomic number of at most 4.

According to a further embodiment, the voltage generator is configured to control the electron source to switch between a generation of the electrons of the first energy and a generation of the electrons of the second energy so that the electrons of the first energy and the electrons of the second energy are successively generated. A time for the switching between the generation of the electrons of the first energy and the generation of the electrons of the second energy is equal to or shorter than 1 millisecond or equal to or shorter than 100 microseconds or equal to or shorter than 10 microseconds. Additionally or alternatively, multiple cathodes may be provided which are charged with different voltages to generate electron beams of different kinetic energies.

According to a further embodiment, the electron receiving member is arranged within a housing of the X-ray source system having an X-ray transmissive portion. A portion of the X-rays, which are generated within the conversion structure traverse a portion of the substrate before exiting from the housing of the X-ray source system through the X-ray transmissive portion. The housing may be configured as an X-ray tube housing.

At least a portion of the X-rays which exit the housing through the X-ray transmissive portion may be incident on the subject to be inspected. A surface portion of the conversion structure on which the electrons are incident may face away from the X-ray transmissive portion of the housing. A surface normal of the surface portion, which points outward from the electron receiving member, may point toward the rotation axis of the electron receiving member.

According to a further embodiment, the substrate comprises an exit surface portion through which the X-rays exit from the substrate before exiting the housing through the X-ray transmissive portion. The electron receiving member comprises a coating which is directly or indirectly applied to at least a portion of the substrate. The coating is configured as a filter and/or an attenuator for the X-rays which are exiting through the exit surface portion. The coating may cover at least a portion of the exit surface portion.

According to a further embodiment, a thickness of the coating varies in at least one lateral direction of the coating (i.e. in a direction perpendicular to a thickness direction of the coating) for providing a spatially varying filtering and/or a spatially varying attenuation of the X-rays for adapting a intensity profile of the X-rays, which exit the housing through the X-ray transmissive portion. The intensity profile of the X-rays may be measured at a position where the X-rays exit from the housing through the X-ray transmissive portion. The intensity profile may be measured in a direction perpendicular to a central ray of the beam formed by the X-rays which exit from the housing through the X-ray transmissive portion. A ratio of a maximum thickness of the coating to a minimum thickness of the coating may be greater than 1.1, or greater than 1.2, or greater than 1.5, or greater than 2, or greater than 5, or greater than 10.

According to a further embodiment, the coating has an X-ray characteristic K-line which is at least partially overlapping with a characteristic K-line of at least a portion of the conversion structure. The characteristic K-line of the coating may coincide with the characteristic K-line of the conversion structure. The coating and at least one layer of the conversion structure may contain one or more same elements. In each of the one or more layers of the conversion structure and the coating, a total amount of the one or more same elements may be at least 50 wt%, or at least 70 wt%, or at least 80 wt%.

According to a further embodiment, the conversion structure comprises at least a first and a second layer. Each of the first and second layers may contain one or more elements in a total amount of at least 50 wt%, or in a total amount of at least 70 wt%, or in a total amount of at least 80 wt%. In the first and in the second layer, each of the one or more elements of the respective layer has an atomic number of at least 29, or at least 42, or at least 74. The one or more elements in the first layer may be the same as, or may be at least partially different from, the one or more elements in the second layer. A chemical composition and/or a crystal structure of the first layer may be different from a chemical composition and/or a crystal structure of the second layer. The term "crystal structure" may be defined herein to mean a description of the ordered arrangement of atoms, ions and/or molecules in a crystalline material. An example for a crystal structure is the body-centered cubic (bcc) crystal structure.

According to a further embodiment, the first layer contains a refractory metal element or an alloy of refractory metal elements. The first layer may contain the refractory metal or the alloy of refractory metal elements in an amount of at least 50 wt%, or at least 70 wt%, or at least 80 wt%. Additionally or alternatively, the second layer contains one or more of tungsten (W), rhenium (Re), tantalum (Ta), tantalum carbide (TaC), molybdenum (Mo) and tungsten carbide (WC). In particular, the second layer may contain one or more of tungsten (W), rhenium (Re), tantalum (Ta), tantalum carbide (TaC), molybdenum (Mo) and tungsten carbide (WC) in a total amount of at least 50 wt%, or at least 70 wt%, or at least 80 wt%. The second layer may be disposed between the first layer and the substrate.

The term "refractory metal element" may be defined to mean one of niobium (Mb), molybdenum (Mo), tantalum (Ta), tungsten (W), rhenium (Re), titanium (Ti), vanadium (V), chromium (Cr), zirconium (Zr), hafnium (Hf), ruthenium (Ru), rhodium (Rh), osmium (Os) and iridium (Ir). The first layer may have a thermal heat conductivity of greater than 100 Wm⁻¹K⁻¹.

Each of the layers of the conversion structure may have a thickness of more than 1 micrometer or more than 2 micrometer. For each of the layers, a maximum thickness of the layer may be less than 20 micrometer or less than 10 micrometer. A thickness ratio of the second layer to the first layer may be greater than 0.5 or greater than 0.7. The thickness ratio may be less than 2 or less than 1.5. The first and the second layers may be adjacent relative to each other. Alternatively, the first and the second layers may be connected via one or more immediate layers. The intermediate layers may form part of the conversion structure or may form part of the electron receiving member without being part of the conversion structure.

According to a further embodiment, the electron receiving member comprises a beam dump. The beam dump may be in a fixed relation relative to the substrate. The beam dump may be arranged radially inward from the conversion structure, as seen relative to the rotation axis of the electron receiving member.

The beam dump may include an electron receiving portion which has no line of sight with the X-ray transmissive portion of the housing and/or with the X-ray detector of the X-ray imaging system. At least a portion of the electron receiving portion may be formed by a coating. The coating may be directly or indirectly applied to the substrate. The coating may include a refractory metal element or an alloy of refractory metal elements in a total amount of at least 50 wt%, or at least 70 wt%, or at least 80 wt%. At least a portion of the beam dump maybe formed as a recess in the electron receiving member.

According to a further embodiment, at least a portion of the substrate contains one or a more of graphite (C), beryllium (Be) and silicon carbide (SiC). The at least the portion of the substrate may contain one or a more of graphite (C), beryllium (Be) and silicon carbide (SiC) in a total amount of at least 50 wt%, or at least 70 wt%, or at least 80 wt%.

Embodiments of the present invention pertain to a computer tomography system which includes the X-ray source system of any one of the preceding claims.

The computer tomography system may be configured for multiple-energy X-ray scanning, in particular for dual-energy X-ray scanning. The computer tomography system may be configured as a multi-energy computer tomography system, in particular as a dual-energy computer tomography system.

### BRIEF DESCRIPTION OF THE DRAWINGS

Figure 1 is a schematic illustration of an X-ray imaging system, which includes an X-ray source system according to one of the exemplary embodiments described herein;
Figure 2 is a schematic cross-sectional view of the X-ray source according to a first exemplary embodiment;
Figure 3 a schematic cross-sectional view of an electron receiving member of the X-ray source system according to the first exemplary embodiment, which is shown in Figure 2;
Figure 4 is a schematic cross-sectional illustration of an electron receiving member of an X-ray source system according to a second exemplary embodiment; and
Figure 5 is a schematic cross-sectional view of an electron receiving member of an X-ray source system according to a third exemplary embodiment.

### DETAILED DESCRIPTION OF EMBODIMENTS

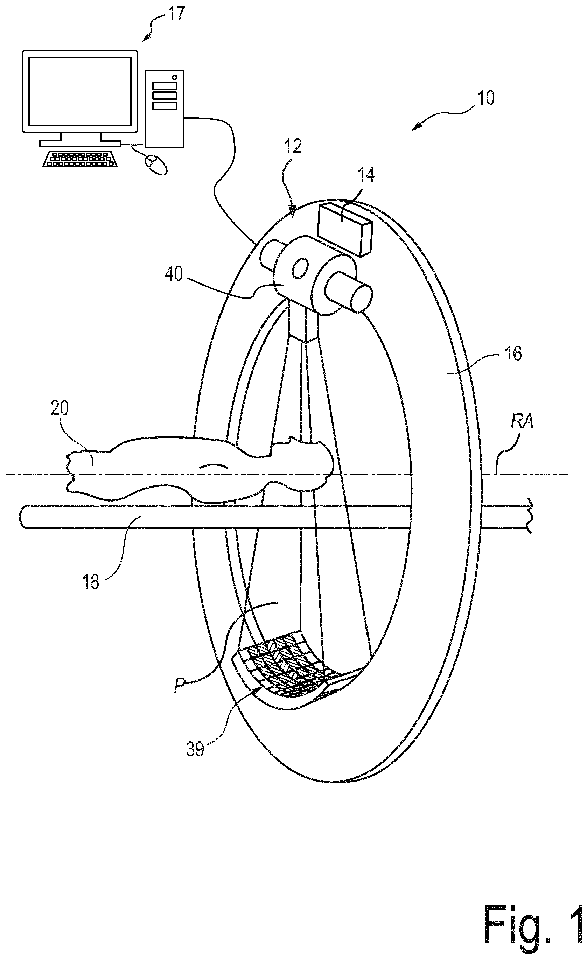

Figure 1 is a schematic illustration of an X-ray imaging system 10, which includes an X-ray source system 12 according to one of the exemplary embodiments described herein. The X-ray imaging system 10 is configured as a computer tomography imaging system, which includes a gantry 16, on which an X-ray source system 12 and the X-ray detector system 39 are mounted on substantially opposite sides relative to a rotation axis RA of the gantry 16 so that the X-ray source system 12 and the detector system 39 are rotatable together with the gantry 16 about a rotation axis RA. The X-ray imaging system 10 further includes table 18 on which the subject 20 may be laid out. The X-ray system further includes a data processing system 17 which receives imaging data acquired from a scan of the subject and which is configured for processing and visualization of the the imaging data.

The X-ray imaging system 10 is configured as a dual-energy computer tomography system, which uses two different X-ray spectra which are emitted from the same X-ray source system (also denoted as "single-source DECT system"). The X-ray source system 12 includes an X-ray tube 40 and a voltage generator 14, which is configured to supply a voltage to the X-ray tube 40 to switch the electron acceleration voltage of the X-ray tube 40 between two voltage values. For each projection, the detector system 39 collects data at each of the two energies. A difference between the first and the second electron energy may be greater than 4 keV, or greater than 10 keV, or greater than 20 keV or greater than 30 keV. Additionally or alternatively, the first energy may be at least 80 keV, or at least 120 keV, or at least 140 keV. The X-ray source system 12 may be configured so that a time for switching between a first operation mode, in which a first acceleration voltage is used, and a second operation mode, in which the second acceleration voltage is used, is equal to or shorter than 1 millisecond or equal to or shorter than 100 microseconds, or equal to or shorter than 10 microseconds.

It is also conceivable, that the voltage generator 14 is configured so that electrons of more than two different electron energies can be generated so that the X-ray source system is switchable between more than two operation modes, each of which being configured to generate electrons of a predefined energy.

By way of example, in the first operation mode, electrons are generated within the X-ray tube 40 having an energy of 80 keV and in a second operation mode, electrons are generated having an energy of 140 keV. For each of these electron energies, an energy distribution of the electrons may have a width, which is less than 2 eV or less than 1 eV.

The different electron energy levels can be used to operate the X-ray imaging system 10 (shown in Figure 1) in a dual-energy imaging mode so that the system is configured as a "fast switching dual-energy computer tomography system". Compared to conventional computer tomography techniques, in which only the distribution of linear attenuation coefficients are obtained, dual-energy computed tomography allows elimination of beam hardening effects and acquisition of information about electron density and atomic number density, which can be used to discriminate between materials with otherwise equal total absorption coefficient.

Specifically, since certain materials or tissues may attenuate X-ray beams differently at different energy levels, spectral evaluation using dual energy computer tomography may be used to accentuate clinically relevant characteristics of the tissue of interest, or to improve differentiation of certain tissues, such as vessels filled with a solution of iodine and bone. Spectral characterization using dual energy computer tomography is highly dependent on the atomic number of tissues. Different tissues attenuate by Compton scattering or photoionization in different ways. For example, many common constituents of normal tissues in the human body have low atomic numbers. Therefore, it would be difficult to distinguish these constituents based on their spectral properties. However, iodine has a much higher atomic number (i.e. Z = 53), which results in a strong spectral contrast between the heavy atoms of iodinated contrast agents used in computer tomography scanning, and the light atoms of the tissues in the body.

However, in conventional dual-energy computer tomography systems, if a same or a similar electron current level is used to generate the different X-ray spectra, this results in different photon fluxes, between the X-ray spectra, since the efficiency of X-ray generation in the target material of the X-ray tube 40 is different for electrons of different kinetic energies.

By way of example, the inventor has shown that, for conventional targets, the X-ray generation efficiency for an electron beam of 140 keV kinetic energy is approximately three times the X-ray generation efficiency for an electron beam of 80 keV kinetic energy. Further, a flux of the photons of the 140 kVp spectrum, measured at a position P downstream of the subject, is typically even seven times the flux of the photons of the 80 kVp spectrum. The abbreviation kVp denotes the temporal maximum of the tube voltage.

The difference in photon fluxes results in different signal to noise ratios of the data sets. This is detrimental for the image quality, as it impairs the angular sampling of the computer tomography system. If the difference in photon flux is compensated by a longer sampling period for photons generated with 80 kVp tube voltage compared to the sampling period for photons generated with 140 kVp tube voltage, this leads to a deteriorated spatial in-plane resolution, in particular for peripheral tissue portions, if sparse sampling schemes are used for processing the data sets.

Generally, it is conceivable to adapt the electron beam current level when switching between the acceleration voltages, so that both X-ray spectra yield substantially a same signal to noise ratio. However, it has been shown by the inventor that this requires a complex and costly design of the voltage generator 14 and the X-ray tube 40, notably the anode.

On the other hand, the inventor has also shown that it is possible to configure the X-ray tube 40 so that the difference in photon flux at the position P downstream of the subject is reduced or even balanced.

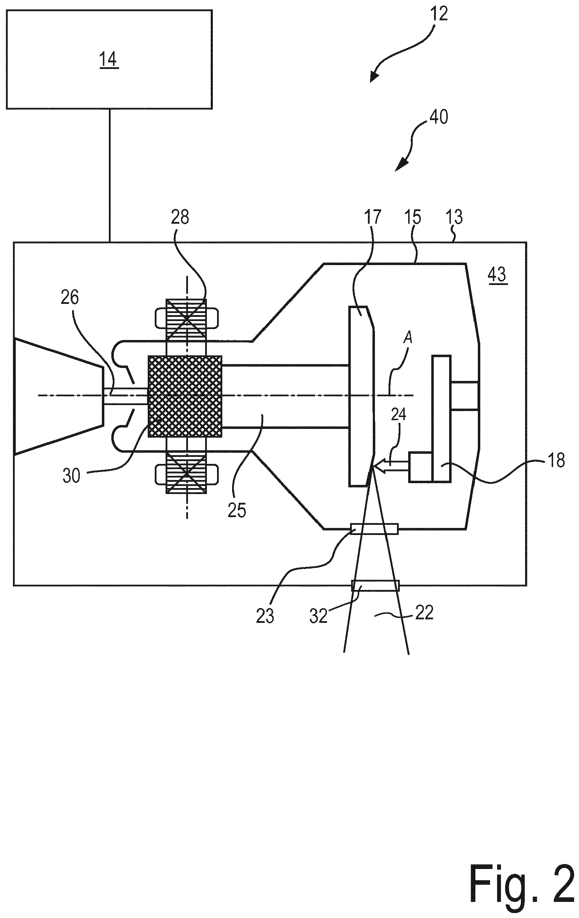

Figure 2 is a schematic view of the X-ray source system 12 according to a first exemplary embodiment. The X-ray source system 12 includes an electron receiving member 17, which is configured as a rotating anode. The X-ray source 12 further includes an electron source 18, which includes a cathode and an electron optical system for generating an electron beam 24, which is directed toward the electron receiving member 17. It is also conceivable that the X-ray source 12 additionally or alternatively includes a grid (not shown in Figure 1), which is disposed between the electron source 18 and the electron receiving member 17 and which provides the functionality of the anode.

The X-ray source 12 includes a housing 13 in which the electron receiving member 17 and at least a portion of the electron source 18 is arranged. The X-ray source system 12 may further include a tube envelope 15, which is arranged within the housing 13 and which provides a vacuum within which the electron receiving member 17 and at least a portion of the electron source 18 is arranged. At least a portion of the space 43 between the housing 13 and the tube envelope 15 maybe filled with an insulating oil.

The electrons of the electron beam 24 are decelerated within the electron receiving member 17, thereby generating bremsstrahlung radiation, which is emitted from the interaction region. The spectral range of the bremsstrahlung radiation has an upper limit (i.e. the cut-off energy of the spectrum), which corresponds to the kinetic energy of the incident electrons. Depending on the energy of the incident electrons and the chemical composition of the portion of the electron receiving member 17, which constitutes the interaction region, the emitted spectrum also includes characteristic radiation. A portion of the generated X-rays traverse a light-transmissive portion 23 of the tube envelope 15 and a light-transmissive portion 32 of the housing 13, thereby forming a beam 22 of X-rays, which is directed toward the subject.

A portion of the kinetic energy of the electrons is dissipated from the interaction region within the electron receiving member 17 as 50 kW to 100 kW of heat energy. For this reason, the electron receiving member 17 is rotated at frequencies as high as 200 Hz to ensure that the target area (i.e. the focal track on the electron receiving member 17) is not damaged by excessive heating.

The X-ray source system 12 may further include a bearing system 25, which is arranged within the tube envelope 15 between a support shaft which supports the electron receiving member 17 and and an outer rotor 26 which is provided outside the tube envelope 15. Typically, the bearing system 25 includes a liquid metal bearing system to enable heat conduction from the rotating electron receiving member 17 out of the vacuum provided by the tube envelope 15. The X-ray source system 12 may also include a motor system, which includes a stator 28, which is attached to the housing 13 and a rotor body 30. By way of example, the rotor body 30 includes a copper cylinder. In operation, energization of the stator 28 causes the electron receiving member 17 to move around a rotation axis A which may be defined by the bearing system 25.

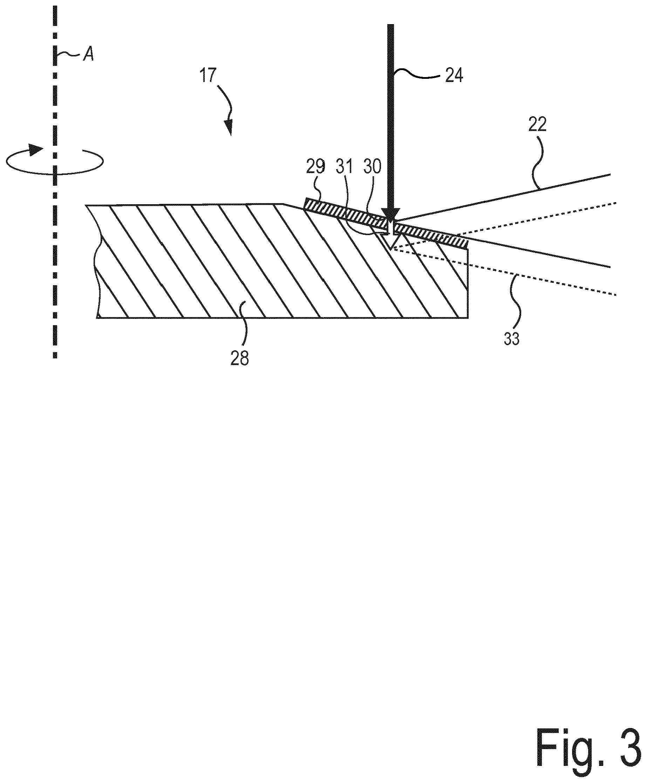

Figure 3 is a schematic cross-sectional view of the electron receiving member 17 of the X-ray source system 12 according to the first exemplary embodiment, which is shown in Figure 2.

The electron receiving member 17 includes a substrate 28 and a conversion structure 29 which includes one or more layers (not shown in Figure 3) which are directly or indirectly applied to the substrate 28. The at least one electron beam 24 is generated by the electron source 18 so that the beam 24 impinges on a surface portion 30 of the conversion structure 29. A portion (schematically illustrated by arrow 31) of the electrons of the electron beam 24 is transmitted through the conversion structure 29 to be subsequently stopped within the substrate 28.

The electron receiving member 17 is configured so that a conversion of the kinetic energy of the electrons into X-rays is suppressed outside of the conversion structure 29 compared to within the conversion structure 29. This is achieved by configuring the conversion structure 29 so that each layer of the one or more layers of the conversion structure 29 has one or more elements in a total amount of at least 50 wt%, or at least 70 wt%, or at least 80 wt%. In each of the layers, each of the one or more elements of the respective layer has an atomic number of at least 29, or at least 42, or at least 74, or at least 75. For each pair of the layers, the one or more elements in the first layer may be the same, or may be at least partially different from the one or more elements in the second layer.

Further, this is achieved by configuring at least a portion of the substrate 28, in which a portion of the electrons are stopped, so that the portion has one or more elements in a total amount of at least 50 wt%, or at least 70 wt%, or at least 80 wt%, wherein each of the elements has an atomic number of at most 13 or at most 6 or at most 4. Thereby, the intensity of the X-ray beam 33, which is formed by the electrons which interact or which are stopped within the substrate 28, is comparatively low. Thereby, only a small amount of off-focal radiation is generated.

Specifically, one or more layers of the conversion structure 29 may contain a refractory metal element or an alloy of refractory metal elements, wherein in each of these layers, the refractory element or the alloy of refractory elements is contained in an amount of at least 50 wt%, or at least 70 wt%, or at least 80 wt%. Additionally or alternatively, at least a portion of the substrate 28, in which at least a portion of the electrons is stopped may contain one or more of graphite, beryllium, and silicon carbide in a total amount of at least 50 wt%, or at least 70 wt%, or at least 80 wt%.

As mentioned before, the electron source 18 is configured to generate, in a first operation mode of the X-ray source system 12, electrons having a first energy and, in a second operation mode of the X-ray source system 12, electrons having a second energy. The first energy is greater than the second energy.

The conversion structure 29 is configured so that, when seen along an axis of the electron beam 24, a ratio of a transmittance for the electrons of the first energy through the conversion structure 29 to a transmittance for the electrons of the second energy through the conversion structure 29 is greater than 1.2 or greater than 1.4 or greater than 1.5 or greater than 2 or greater than 2.5, or greater than 3, or greater than 5, or greater than 10.

Thereby, a greater portion of the electrons having the first energy traverse the conversion structure 29 and are stopped within the substrate 28 compared to the electrons having the second energy. Hence, a greater portion of the electrons having the second energy (i.e. the electrons having the lower energy) is stopped within the conversion structure 29. It has been shown by the inventor that this allows compensating different conversion efficiencies for converting the kinetic electron energy into X-rays between the electrons of the first energy and the electrons of the second energy. Such a compensated difference in conversion efficiency allows acquisition of data during a CT scan without having a significant variation in the signal to noise ratio between data acquired at different energy levels. This allows evaluation of the dual-energy computer tomography data so that a high spatial resolution is obtained.

Specifically, by using the conversion structure, as described above, it is possible to adapt the ratio of the photon flux of the 140kVp photons compared to the photon flux of the 80 kVp spectrum so that at the position P (shown in Figure 1) downstream of the subject, the difference in photon flux is reduced or even adapted to be substantially equal.

In some embodiments of the present disclosure, the desired range for the ratio of the transmittance for the electrons having the first energy to the transmittance for the electrons having the second energy is achieved using a conversion structure 29 having a thickness, as measured in a direction parallel to an axis of the electron beam 24, which is less then 20 micrometer or less than 10 micrometer or less than 7 micrometer. The conversion structure 29 may have a thickness, measured in a direction parallel to the axis of the electron beam 24, which is greater than 0.5 micrometer or greater than 1 micrometer.

The conversion structure 29 may have a layer, wherein the layer contains a refractory metal element or an alloy of refractory metal elements in an amount of at least 50 wt%, or at least 70 wt%, or at least 80 wt%.

The conversion structure may be a multilayer structure which includes at least a first and a second layer, each of which including one or more elements in a total amount of at least 50 wt%, or at least 70 wt%, or at least 80 wt%. In the first and the second layer, each of the one or more elements of the respective layer has an atomic number of at least 29 or at least 42 or at least 74 or at least 75. The one or more elements in the first layer may be the same as, or may be at least partially different from, the one or more elements in the second layer.

A chemical composition and/or a crystal structure of the first layer may be different from a chemical composition and/or a crystal structure of the second layer.

If the conversion structure 29 is a multilayer structure, the topmost layer may have a refractory metal element or an alloy of refractory metal elements in an amount of at least 50 wt%, or at least 70 wt%, or at least 80 wt%.

Specifically, since the topmost layer of the conversion structure 29 is directly exposed to the incident electron beam, this can lead to temperatures on the top surface of the conversion structure 29 in excess of 2,500 °C. Therefore, providing a refractory metal element or an alloy of refractory metal elements in an amount of at least 50 wt%, or at least 70 wt%, or at least 80 wt% in the topmost layer improves the lifetime of the focal track and enables heat to be dissipated more effectively. The refractory metal element or the alloy of refractory metal elements may be selected so that the topmost layer of the conversion structure has a thermal conductivity, which is greater than 100 Wm⁻¹K⁻¹.

The conversion structure 29 may include a second layer which is arranged between the first layer and the substrate 28, wherein the first layer has a refractory metal element or an alloy of refractory metal elements in an amount of at least 50 wt%, or at least 70 wt%, or at least 80 wt%. According to an embodiment, the second layer includes one or more of tungsten (W), rhenium (Re), tantalum (Ta), tantalum carbide (TaC), molybdenum (Mo) or tungsten carbide (WC) in a total amount of at least 50 wt%, or at least 70 wt%, or at least 80 wt%.

The first layer and the second layer may be adjacent to each other or may be separated from each other by one or more intermediate layers. The intermediate layers may be part of the conversion structure 29 or may have a material as main constituent, which has a comparatively low atomic number so that the intermediate layers are not part of the conversion structure 29. It has been shown by the inventor that the combination of the first and second layer, as described above, leads to an improved mechanical resilience and an improved thermal dissipation. This, in turn, allows reduction of the thickness of the layers of the conversion structure 19. It has further been shown by the inventor that the reduced thickness allows adaptation of the difference between the photon flux of the X-rays generated by the high-energy electrons and the photon flux generated by the low-energy electrons so that the difference in the photon flux at a position P (shown in Figure 1) downstream of the subject is reduced to a predefined level.

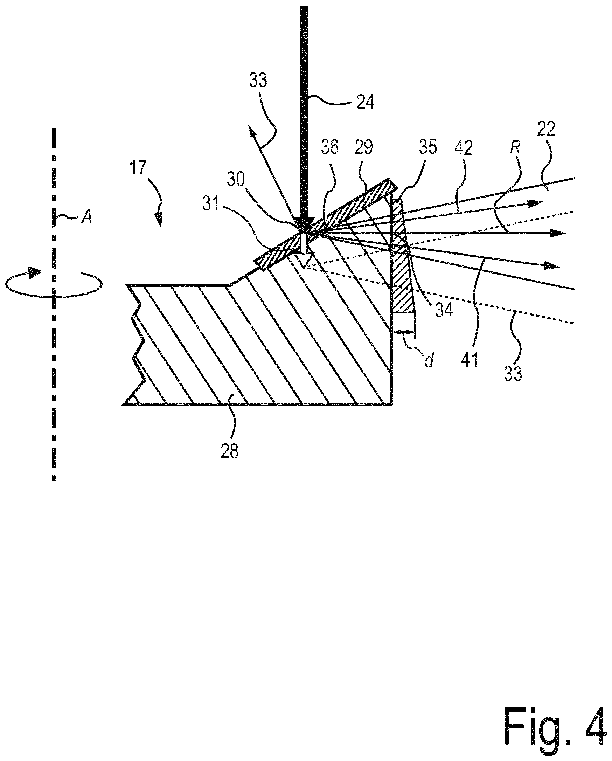

Figure 4 is a schematic cross-sectional view of an electron receiving member 17 of an X-ray tube system 12 according to a second exemplary embodiment and showing a transparent target construction. In the electron receiving member 17 of the second exemplary embodiment, the surface portion 30 of the conversion structure 29 upon which the electrons of the electron beam 24 are incident, faces away from the X-ray transmissive portions of the housing and the tube envelope (denoted with reference numerals 23 and 32 in the first embodiment, which is shown in Figure 2). A surface normal vector 33 of the surface portion 30, which points outward from the electron receiving member 17 may point toward the rotation axis A of the electron receiving member 17. Thereby, the X-rays, which are generated within the conversion structure 29 and which exit the tube through the X-ray transmissive portion of the housing, traverse a portion of the substrate 28, after having exited from the conversion structure 29. In particular, X-rays, which have been generated within the conversion structure 29, pass through an interface 36 between the conversion structure 29 and the substrate 28, then through a portion of the substrate 28 before traversing the X-ray transmissive portion of the housing.

The inventor has shown that thereby, the X-ray transmissive portions of the tube envelope and the housing (denoted with reference numerals 23 and 32 in the first exemplary embodiment, which is shown in Figure 2) are shielded from at least a portion of the back scattered electrons and secondary electrons, which are generated by the impact of the electron beam 24 on the conversion structure 29.

Further, the inventor has shown that this configuration of the substrate 28 of the X-ray receiving member according to the second exemplary embodiment allows application of a coating 35 to an exit surface portion 34 of the substrate 28, wherein through the exit surface portion 34, the X-rays exit from the substrate 28. In the exemplary embodiment, the exit surface portion 34 is an outer circumferential surface portion of the substrate 28. The coating 35 is configured to acts as a filter and/or an attenuator for the X-rays and is in the X-ray beam path between the substrate 28 and the X-ray transmissive portion of the housing.

By way of example, the filter may absorb a portion of the X-rays which exit from the substrate 28 through the exit surface portion 34 so that a spectrum of the X-rays, which are incident on the X-ray transmissive portion of the tube envelope is different compared to a spectrum of X-rays which exit from the substrate 28 through the exit surface portion 34. Thereby, the coating 35 acts as a filter. Additionally or alternatively, the coating 35 may generate, decrease, or increase one or more lines of characteristic radiation. By way of example, the coating 35 may have a characteristic K-line, which is at least partially overlapping with a characteristic K-line of at least a portion of the conversion structure 29. Each of the coating 35 and at least one layer of the conversion structure 29 may contain one or more same elements. In each of these layers, a total amount of the one or more same elements may be at least 50 wt%, or at least 70 wt%, or at least 80 wt%. Additionally or alternatively, the coating 35 may have a refractory metal element or an alloy of refectory metal elements in an amount of at least 50 wt%, at least 70 wt%, or at least 80 wt%.

Additionally or alternatively, the coating 35 may absorb a portion of the X-rays without significantly modifying the spectrum so that the coating substantially acts as an attenuator. Examples for suitable attenuation materials are, but are not limited to one or more of: tungsten (W), rhenium (Re), molybdenum (Mo), tantalum (Ta) and chemical compounds of these elements with other elements. Notably, the attenuating layer may have a same or similar composition as at least a portion of the X-ray conversion layer. This allows mimicking the intrinsic absorption characteristics of at least the portion of the conversion layer under the different electron interactions.

The filtering and/or attenuation of the coating 35 may vary spatially, for example, caused by a varying thickness d of the coating. By way of example, the coating 35 may be configured as a spatially varying attenuator and/or filter so that the intensity profile in a direction perpendicular or substantially perpendicular to a direction of a central ray R of the X-ray beam can be made more homogeneous. By way of example, the intensity profile may be measured at a position where the X-rays exit from the housing through the X-ray transmissive portion of the housing.

In particular, as is illustrated in Figure 4, a thickness d of the coating 35 may decrease with decreasing distance from the electron source. Thereby, the spatially varying filter and/or attenuator is configured to more strongly attenuate X-rays 41, which have a comparatively short optical path length through the conversion structure 29 and thereby are less attenuated by the conversion structure 29, compared to other X-rays 42, which have a comparatively large optical path length through the conversion structure 29 and are therefore more strongly attenuated by the conversion structure.

It is also conceivable that the coating 35 is configured to substantially block the X-rays 42, which have a comparatively large optical path length through the conversion structure 29 and not to block the X-rays 41, which have a comparatively short optical path length through the conversion structure 29. By way of example, the coating 35 may extend only over a portion of the exit surface portion 34 so that only a portion of the X-rays which exit from the substrate 28 through the exit surface portion 34 are blocked.

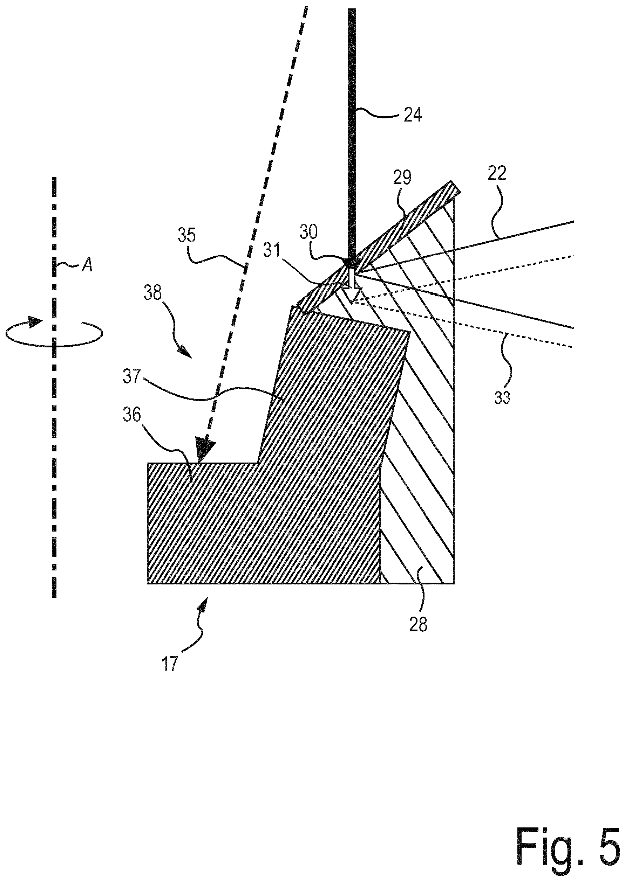

Figure 5 is a schematic cross-sectional view of an electron receiving member 17 of an X-ray source system according to a third exemplary embodiment. In a similar manner as the electron receiving member 17 of the second exemplary embodiment, which is illustrated in Figure 4, the surface portion 30 upon which the electrons of the electron beam 24 are incident, faces away from the X-ray transmissive portions of the housing and the tube envelope. However, it is also conceivable that the surface portion 30 faces toward the X-ray transmissive portions of the housing and the tube envelope, as is, for example, illustrated for the first exemplary embodiment in Figure 3.

The electron receiving member 17 of the third exemplary embodiment includes a beam dump 38 which is arranged radially inward of the conversion structure 29, when seen relative to the rotation axis A of the electron receiving member 17. Thereby, it is possible to switch off generation of the X-rays by deflecting the beam 24 radially inward, i.e. in a manner, as is illustrated by arrow 35, so that the electrons are incident on an electron receiving portion 36 of the beam dump 38, which has no line of sight with the X-Ray transmissive portions of the housing and the tube envelope. In the electron receiving portion 36, the electrons of the electron beam 25 are received and stopped.

Additionally, the beam dump may include a shielding portion 37 which is disposed radially outward relative to the electron receiving portion 36. The shielding portion 37 may be configured to shield at least a portion of the X-ray transmissive portion of the housing and/or the X-ray transmissive portion of the tube envelope from X-rays and/or electrons which are generated by the impact of the electron beam on the electron receiving portion 36.

By way of example, the electron receiving portion 36 and/or the shielding portion 37 may contain, in a total amount of at least 50 wt%, or at least 70 wt%, or at least 80 wt%: carbon, beryllium and/or one or more materials which sustain high power density under electron bombardment.

The above embodiments as described are only illustrative, and not intended to limit the technique approaches of the present invention. Although the present invention is described in details referring to the preferable embodiments, those skilled in the art will understand that the technique approaches of the present invention can be modified or equally displaced without departing from the protective scope of the claims of the present invention. In the claims, the word "comprising" does not exclude other elements or steps, and the indefinite article "a" or "an" does not exclude a plurality. Any reference signs in the claims should not be construed as limiting the scope.

## Claims

1. An X-ray source system (12) configured for successively or concurrently generating different X-ray spectra, which have different cutoff-energies, the system comprising:
an electron receiving member (17), which is mounted to be rotatable about a rotation axis (*A*);
wherein the electron receiving member (17) comprises a conversion structure (29) and a substrate (28), wherein the conversion structure (29) comprises one or more layers which are directly or indirectly applied to the substrate (28);
an electron source (18) configured to, successively or concurrently, generate one or more beams (24) of electrons which are incident on the conversion structure (29);
a voltage generator (14), which is in signal communication with the electron source (18) and which is configured to control the electron source (18) so that electrons having a first energy and a second energy are successively or concurrently generated using different acceleration voltages of the electron beam source (18);
wherein within the electron receiving member (17), a conversion of a kinetic energy of the electrons into X-rays is suppressed outside of the conversion structure (29) compared to within the conversion structure (29); and
wherein the first energy is greater than the second energy and a ratio of a transmittance for the electrons of the first energy through the conversion structure (29) to a transmittance for the electrons of the second energy through the conversion structure (29) is greater than 1.2 or greater than 1.4 or greater than 1.5.

2. The X-ray source system (12) of claim 1, wherein at a portion of the conversion structure (29), where the electrons of the first and second energies are incident, a thickness of the conversion structure (29), as measured in a direction parallel to an axis of at least one of the one or more electron beams (24), is less than 20 micrometer, or less than 10 micrometer, or less than 7 micrometer.

3. The X-ray source system (12) of claim 1 or 2, wherein the voltage generator (14) is configured to control the electron source (18) to switch between a generation of the electrons of the first energy and a generation of the electrons of the second energy so that the electrons of the first energy and the electrons of the second energy are successively generated;
wherein a time for the switching between the generation of the electrons of the first energy and the generation of the electrons of the second energy is equal to or shorter than 1 millisecond, or equal to or shorter than 100 microseconds, or equal to or shorter than 10 microseconds.

4. The X-ray source system (12) of any one of the preceding claims wherein the conversion structure (29) comprises at least a first and a second layer, each of which containing one or more elements in a total amount of at least 50 wt%,
wherein in the first and the second layer, each of the one or more elements of the respective layer has an atomic number of at least 29 or at least 42 or at least 74;
wherein a chemical composition and/or a crystal structure of the first layer is different from a chemical composition and/or a crystal structure of the second layer.

5. The X-ray source system (12) of claim 4, wherein:
the first layer contains a refractory metal element or an alloy of refractory metal elements in an amount of at least 50 wt%; and
the second layer includes one or a more of tungsten (W), rhenium (Re), tantalum (Ta), tantalum carbide (TaC), molybdenum (Mo) and tungsten carbide (WC) in a total amount of at least 50 wt%.

6. The X-ray source system (12) of any one of the preceding claims, wherein for each of the one or more layers of the conversion structure (29), the respective layer comprises one or more elements in a total amount of at least 50 wt%,
wherein in each of the one or more layers, each of the one or more elements of the respective layer has an atomic number of at least 29, or at least 42, or at least 74, or at least 75.

7. The X-ray source system (12) of any one of the preceding claims, wherein at least a portion of the substrate (28), within which at least a portion of the electrons are stopped, comprises one or more elements in a total amount of at least 50 wt%, wherein each of the one or more elements has an atomic number of at most 13, or at most 6, or at most 4.

8. The X-ray source system (12) of any one of the preceding claims, wherein:
a difference between the first and the second energy is greater than 4 keV, or greater than 10 keV, or greater than 20 keV or greater than 30 keV; and/or
the first energy is at least 80keV, or at least 120keV, or at least 140keV.

9. The X-ray source system (12) of any one of the preceding claims,
wherein the electron receiving member is arranged within a housing (13) of the X-ray source system (12) having an X-ray transmissive portion (32); and
wherein a portion of the X-rays which are generated within the conversion structure (29) traverse a portion of the substrate (28) before exiting from the housing (13) through the X-ray transmissive portion (32).

10. The X-ray source system (12) of claim 9, wherein the substrate (28) comprises an exit surface portion (34) through which the X-rays exit from the substrate (28) before exiting the housing (13) through the X-ray transmissive portion (32);
wherein the electron receiving member (17) comprises a coating (35) which is directly or indirectly applied to at least a portion of the substrate (28);
wherein the coating (35) is configured as a filter and/or an attenuator for the X-rays which are exiting through the exit surface portion (34).

11. The X-ray source system (12) of claim 10, wherein a thickness of the coating (35) varies in at least one lateral direction of the coating (35) for providing a spatially varying filtering and/or a spatially varying attenuation of the X-rays for adapting an intensity profile of the X-rays which exit the housing (13) through the X-ray transmissive portion (32).

12. The X-ray source system (12) of claim 10 or 11, wherein the coating (35) has at least one characteristic K-line which is at least partially overlapping with a characteristic K-line of at least a portion of the conversion structure (29).

13. The X-ray source system (12) of any one of the preceding claims, wherein the electron receiving member (17) comprises a beam dump (38), which is in a fixed relation relative to the substrate (28) and which is located radially inward from the conversion structure (29), as seen relative to the rotation axis (*A*).

14. The X-ray source system (12) of any one of the preceding claims, wherein at least a portion of the substrate (28) contains one or a more of graphite (C), beryllium (Be) and silicon carbide (SiC) in a total amount of at least 50 wt%.

15. A computer tomography system (10) comprising the X-ray source system (12) of any one of the preceding claims.
